(19) Europäisches Patentamt
European Patent Office
Office européen des brevets

(11) EP 1 562 639 B1

(12) **EUROPEAN PATENT SPECIFICATION**

(45) Date of publication and mention of the grant of the patent:
**18.04.2007 Bulletin 2007/16**

(21) Application number: **03790942.1**

(22) Date of filing: **29.08.2003**

(51) Int Cl.:
***A61K 49/06*** *(2006.01)*

(86) International application number:
**PCT/EP2003/009589**

(87) International publication number:
**WO 2004/019996 (11.03.2004 Gazette 2004/11)**

(54) **METHOD AND ARRANGEMENT FOR PRODUCING CONTRAST AGENT FOR MAGNETIC RESONANCE IMAGING**

VERFAHREN UND VORRICHTUNG ZUM HERSTELLUNG VON KONTRASTMITTELN FÜR MAGNETRESONANZBILDGEBUNG

PROCEDE ET SYSTEME PERMETTANT DE PRODUIRE UN AGENT DE CONTRASTE POUR L'IMAGERIE PAR RESONANCE MAGNETIQUE

(84) Designated Contracting States:
**AT BE BG CH CY CZ DE DK EE ES FI FR GB GR HU IE IT LI LU MC NL PT RO SE SI SK TR**

(30) Priority: **29.08.2002 GB 0219953**

(43) Date of publication of application:
**17.08.2005 Bulletin 2005/33**

(73) Proprietor: **GE Healthcare AS**
**0485 Oslo (NO)**

(72) Inventors:
- **ARDENKJAER-LARSEN**
  **PO Box 4220 Nydalen-N-0401 OSLO (NO)**
- **AXELSSON, Oskar**
  **PO Box 4220 Nydalen N-0401 Oslo (NO)**
- **GOLDMAN, Maurice**
  **F-91140 Villebon sur Yvette (FR)**
- **JOHANESSON, Haukur**
  **PO Box 4220 Nydalen,N-0401 Oslo (NO)**

(74) Representative: **Franks, Barry Gerard et al**
**GE Healthcare Limited**
**Amersham Place**
**Little Chalfont,**
**Bucks. HP7 9NA (GB)**

(56) References cited:
**WO-A-00/40988** **WO-A-00/71166**
**WO-A-99/24080**

- **GOLMAN K ET AL: "Parahydrogen-induced polarization in imaging: Subsecond 13/C angiography." MAGNETIC RESONANCE IN MEDICINE, JULY 2001, WILEY, USA, vol. 46, no. 1, pages 1-5, XP002264070 ISSN: 0740-3194**
- **BARKEMEYER J ET AL: "Heteronuclear polarization transfer using selective pulses during hydrogenation with parahydrogen" JOURNAL OF MAGNETIC RESONANCE, SERIES A, MAY 1996, ACADEMIC PRESS, USA, vol. 120, no. 1, pages 129-132, XP002264071 ISSN: 1064-1858**

EP 1 562 639 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to an apparatus and method for para-hydrogen induced hyperpolarization of a compound, and particularly for the preparation of a contrast agent for magnetic resonance imaging procedures.

## Background of the Invention

Magnetic Resonance Imaging (MRI) is an important diagnostic technique. It is especially attractive since it is non-invasive and does not expose the patient to potentially harmful radiation such as X-rays or radiation from radioactive materials. Significant progress has recently been made in the quality of images as well as finding new applications of the technique. The progress relies on a rapid development of the digital image processing, the refined Nuclear Magnetic Resonance (NMR) techniques and the development of effective contrast agents (imaging agents). An emerging technique of particular interest involves Magnetic Resonance (MR) contrast agents based on the principle of pre-polarization of the nuclear spins, also called hyperpolarization

For the resonance phenomena, which is the basis of NMR and MRI, to occur, isotopes with non-zero nuclear spin have to be present. In addition, since NMR is not an extremely sensitive technique, a relatively high concentration and/or a high gyromagnetic ratio is needed, especially for imaging purposes. The use of a selected isotope in a contrast agent which is to be injected in a patient, puts further requirements on the selected isotope, for example regarding toxicity. A number of isotopes have the required spin properties, but only a few are considered interesting for the use in contrast agents, for example the carbon isotope $^{13}C$ and the nitrogen isotope $^{15}N$. The carbon isotope $^{13}C$ has many properties that would it make it useful as a functional part of an MRI contrast agent. An important feature is the long longitudinal relaxation time, $T_1$. The relaxation time needs to be long in order to have time, after the generation of the contrast agent, to inject the contrast agent into the patient and to allow the contrast agent to be transported to the organ that is to be studied. To make a useful MR-contrast agent of this kind, the signal strength has to be boosted significantly over the thermal equilibrium signal. Patent application WO 00/71166, by the same applicant, describes a process and an apparatus for increasing the polarization and hence the signal from a small organic molecule containing for example $^{13}C$. The signal was increased with a factor $10^4$. The process is referred to as Para-Hydrogen Induced Polarisation (PHIP) and may comprise the transferring of nuclear spin-order from para-hydrogen to spin polarization in non-zero spin nuclei in molecules, e.g. to $^{13}C$ or $^{15}N$ nuclei.

Hydrogen molecules exist in four different spin states. In one of the forms, characterised by antiparallel spins, the magnetic moments of the protons cancel. This form is called para-hydrogen. The other three forms, with a net magnetic moment, are referred to as ortho-hydrogen. The para-hydrogen will not rotate at low temperature whereas the ortho-form must rotate with a high frequency at all temperatures because of quantum-mechanical symmetry requirements of the wave function. This indicates that at low temperature the para-form will have a significantly lower energy, and hence is the energetically favoured form. At temperatures below 20 K the equilibrium ratio of para- and ortho-hydrogen approaches 100:0, at 80 K the ratio is 48:52 and at room temperature approximately 1:3. The equilibration can be speeded up by the presence of a transition metal catalyst, e.g. $Fe_2O_3$. Para-hydrogen relaxes slowly (if no catalyst is present) at room temperature.

In WO 00/71166 it is described how to catalytically hydrogenate (with para-hydrogen) unsaturated compounds comprising non-zero spin nuclei such as $^{13}C$. The spin correlation of the protons from the para-hydrogen will be preserved during and after hydrogenation, and the influence on the spins of the $^{13}C$ nuclei breaks the symmetry of the spin system. The protons will now give an NMR-signal, but the non-equilibrium spin order is not sufficient to make the molecule useful for imaging purposes since it has an anti-phase behaviour that is not ideal for imaging. In the above cited applications, and further in "Parahydrogen-Induced Polarization in Imaging" by K. Golman et al., Magnetic Resonance in Medicine 46:1-5 (2001), a magnetic field cycling method is described for transforming the proton spin-order to carbon spin polarization. In a first step the external magnetic field is reduced (from the relatively high geomagnetic field) bringing the combined proton-carbon spin system into its strong coupling regime. In this regime the scalar coupling (J-coupling) strongly influences the evolution of the spin system. The reduction of the field should be fast, giving a diabatic (non-adiabatic) process. In a subsequent step the field strength is slowly increased (an adiabatic process). The field cycling will result in a substantial increase in the polarization of the spins of the $^{13}C$ nuclei, giving an in-phase NMR-signal, and increase the usefulness of the compound as a contrast agent for use in imaging procedures. However, the result of the field treatment will be dependent on the scalar coupling of the combined spin system and the properties of the magnetic field. In WO 00/71166 examples of field cycling schemes are described that give a substantial improvement in the image quality. To make the method even more attractive for use in medical and diagnostic applications it would be of high value to further increase the degree of polarisation of the imaging nuclei spins (e.g. $^{13}C$ or $^{15}N$ nuclei) and shorten the production times for PHIP contrast agents.

## Summary of the Invention

The object of the present invention is to provide a method and an apparatus for producing MRI contrast agent with a high degree of polarisation of the imaging nuclei spins with a short production time.

The object is achieved by the method as defined in claims 1 and 10, the apparatus as defined in claim 14, and by the computer program product as defined in claims 12 and 13.

The method for producing contrast agent according to the present invention comprises the steps of obtaining a solution in a solvent of a hydrogenatable, unsaturated substrate compound and a catalyst for the hydrogenation of a substrate compound, hydrogenating the substrate with hydrogen gas ($H_2$) enriched in para-hydrogen (p-$^1H_2$) to form a hydrogenated contrast agent and exposing the contrast agent to a magnetic field cycling profile adapted for enhancing the contrasting effects of the contrast agent. The magnetic field cycling profile comprises an initial decrease of the magnetic field followed by at least one increase of the magnetic field, which increase should be arranged as to give a non-adiabatic (diabatic) re-magnetisation of the contrast agent.

According to one embodiment of the present invention the magnetic field cycling profile is determined by a process which comprises the steps of finding the quantum mechanical density operator describing the initial spin order of the combined para-hydrogen and imaging nuclei spin system, simulating the polarisation for the imaging nuclei given a field cycling profile and varying the field cycling parameters according to an optimizing routine. The simulating step and varying step should preferably be repeated until the net polarisation reaches a maximum value or a desired value.

The apparatus for producing MR contrast agent according to the present invention comprises a magnetic field screen arranged around a magnetic treatment chamber, and a demagnetization circuit adapted for demagnetization of the magnetic field screen. The demagnetization circuit comprises of a demagnetization coil of about 30 turns arranged on the magnetic shield, a second coil of about 1000 turns and a dipolar capacitor of approximately 250 μF, which together with the second coil forms a parallel resonance circuit connected to the demagnetization coil. The resonance circuit is preferably arranged to have a resonance frequency of approximately 50 Hz.

One advantage afforded by the apparatus and method according to the present invention is that a contrast agent can be produced that significantly improves the image quality and/or speeds up the process in an MRI application and/or improves the analysis performance in an MNR application.

A further advantage is that novel types of imaging, not possible, or very difficult to perform with prior art techniques, can be performed.

The advantages are achieved by the apparatus and method according to the present invention by providing a high degree of polarization of the imaging nuclei of the contrast agent and by that the process of producing contrast agent is fast, i.e. the field cycling profile is fast, thereby reducing the problems with relaxation of the spins system of the contrast agent.

## Brief Description of the Drawings

The invention will now be described in detail with reference to the drawing figures, in which

Fig. 1 is a schematic drawing illustrating the prior art apparatus;

Fig. 2a and 2b are graphs representing probability amplitudes $\left|C_1^\alpha(t)\right|^2$ (dotted), $\left|C_2^\alpha(t)\right|^2$ (dashed) and $\left|C_3^\alpha(t)\right|^2$ (solid) at $B_0$ = 1 μT (a) and $B_0$ = 0.1 μ T (b);

Fig. 3 is a graph representing the carbon polarization during a field cycling;

Fig. 4 is a flowchart describing the method according to the present invention;

Fig 5 is a graph representing an example of an optimized field cycling profile according to the invention;

Fig 6 is a graph representing the polarization during the optimized field cycling profile of figure 5: and

Fig. 7 is a flowchart describing a magnetic treatment according to the present invention.

## Detailed Description of the invention

The apparatus and parts of the process described in WO 00/71166 is advantageously utilised also in the present

invention. WO 00/71166 teaches that the hydrogenation reaction is preferably performed by mixing gaseous para-hydrogen (or ortho-deuterium) enriched hydrogen with a solution of an unsaturated compound and a hydrogenation catalyst.

The present invention relates to a process which comprises the following main steps:

100: obtaining a solution comprising a solvent, a hydrogenatable, unsaturated substrate compound and a catalyst for the hydrogenation of a substrate compound;

105: introducing the solution into a chamber containing hydrogen gas ($H_2$) enriched in para-hydrogen (p- $^1H_2$) to hydrogenate the substrate to form a hydrogenated contrast agent;

The method according to the present invention introduces a main step of

110: exposing the contrast agent to a low-field magnetic treatment comprising an optimized magnetic field cycling profile arranged to provide a non-adiabatic (diabatic) re-magnetisation of the contrast agent.

The optimized field cycling process enables polarization to be transferred from protons in the freshly hydrogenated contrast agent to a nucleus within the same molecule with a longer spin-order life-time, preferably a $^{13}C$ or $^{15}N$ nucleus. These nuclei will be referred to as imaging nuclei. The timing of this process, the precise field profile and the field strength used during this process is critical and is the subject of the present invention. A method of designing very effective field cycling procedures, i.e. producing contrast agent with a high degree of polarization, in accordance with the present invention, will be described below. The method and apparatus will be exemplified with contrast agent comprising carbon ($^{13}C$). This should be regarded as a non limiting example. Other isotopes could be used with slight modifications to the detailed steps of the method.

The hydrogenatable substrate used may be a material such as is discussed in WO99/24080, e.g. a para-hydrogenation substrate. For *in vitro* or *in vivo* MR studies of biological or quasi-biological processes or synthetic polymer (e.g. peptide, polynucleic acid etc.) syntheses, the substrate is preferably hydrogenatable to form a molecule participating in such reactions, e.g. an amino acid, a nucleic acid, a receptor-binding molecule, etc., either a natural such molecule or an analog.

The solvent used in step 100 of a process in accordance with the present invention may be any convenient material which serves as a solvent for the substrate and the hydrogenation catalyst - a number of possible solvents are discussed in WO99/24080. When the contrast agent is for use *in vivo* MR investigations, the solvent is preferably physiologically tolerable. Water is a preferred choice of solvent, used in combination with a water-soluble catalyst. If other solvents that are not physiologically tolerable are used, the solvents has to be removed before use in a patient, for example by vacuum-spray. Other rapid solvent removal techniques, e.g. affinity techniques, may however be used. The solvent is preferably used at or near the minimum quantities required to maintain substrate, catalyst and contrast agent in solution without experiencing viscosity problems during the hydrogenation reaction.

The hydrogenation catalyst is preferably a catalyst of the type discussed in WO99/24080, e.g. a metal complex, in particular a rhodium complex.

The enriched hydrogen, which may be pure $^1H_2$ or $^2H_2$, or a mixture of $^1H_2$ and $^2H_2$, may optionally contain other gases, but is preferably free from oxygen or other reactive or paramagnetic gases, may be prepared by cooling hydrogen, preferably to a temperature below 80 K, more preferably to below 50 K, even more preferably to below 30 K and especially preferably to below 22 K, and allowing the nuclear spin states to equilibrate, optionally in the presence of a solid phase equilibration promoter, e.g. $Fe_3O_4$, $Fe_2O_3$, activated charcoal, etc. The enriched hydrogen is then preferably removed from the equilibrator and optionally stored before use. A method of preparation and storage of enriched hydrogen is described in WO99/24080. A preferred and novel method of storage and equipment for that purpose has been developed by the inventors. The enriched hydrogen is transferred to and stored in gas cylinders made of inert material. Inert in this context should be understood as made of material essentially free from paramagnetic materials (primarily iron) and other para-hydrogen relaxing compounds (e.g. palladium). Examples of inert materials suitable for the gas cylinders are aluminum and carbon fiber reinforced epoxy. In such cylinders the enriched hydrogen will decay slowly (in the order of 10% per week). Such a decay rate is acceptable in most applications, especially compared with the cost and handling problems of previous storing methods involving storing at cryogenic temperatures.

For the hydrogenation reaction, a reaction chamber is filled with enriched hydrogen, optionally under pressure - preferably 5-20 bar, and the catalyst and substrate solution is introduced either as a thin jet, by spraying or by atomising, into this reactor. If desired, the solution may be produced by mixing separate solutions of catalyst and of substrate. To ensure proper mixing, a distributor or a plurality of spray nozzles may be used and the chamber contents may be mixed, e.g. by a mechanical stirrer or by appropriately shaping the chamber walls to cause turbulent mixing when there is a flow of reaction mixture in the chamber.

The process may be performed continuously with a flow reactor, e.g. a loop or tube reactor, or alternatively it may be a batch-wise process. Preferably however there will be a continuous or pulsed flow of enriched hydrogen and solution into the reactor, a continuous or batch-wise removal of liquid solution from the base of the reactor, and a continuous or batch-wise venting of unreacted gas from the reactor. The enriched hydrogen and solution passing into the reactor are preferably temperature-controlled to ensure that the gas/droplet phase in the reactor is at the desired temperature. This can be achieved by providing input lines with temperature sensors and heating and/or cooling jackets.

If a non-aqueous solution has been used, the contrast agent is preferably mixed with water after the hydrogenation and the low magnetic field treatment. The water used is preferably sterile and also preferably essentially free of paramagnetic contaminants. The resultant aqueous solution is then preferably treated to remove the hydrogenation catalyst, e.g. by passage through an ion exchange column, preferably one free of paramagnetic contaminants. The water may be temperature-controlled. The water and contrast agent solutions are mixed in a, preferably temperature-controlled, mixing chamber so as to ensure that the aqueous solution enters the ion exchange column at the appropriate temperature. Strongly acidic, sodium ion charged ion exchange resins such as DOWEX 1x2-400 (Dow Chemicals) and Amberlite IR-120 (both available from Aldrich Chemicals) resins may conveniently be used for the removal of typical metal complex hydrogenation catalysts. For fast ion exchange, the resin is preferably cross-linked to only a low degree, e.g. a 2% divinyl benzene cross-linked sulphonated, sodium ion loaded polystyrene resin.

Removal of the non-aqueous solvent may then conveniently be effected by spray flash distillation e.g. by spraying the aqueous solution into a chamber, applying a vacuum, and driving the organic solvent-free aqueous solution from the chamber using an inert, preferably non-paramagnetic gas, e.g. nitrogen. Indeed in general the flow of liquid components through the hydrogenation apparatus are preferably effected using applied nitrogen pressure, e.g. 2 to 10 bar.

The resulting aqueous contrast agent solution may be frozen and stored or may preferably be used directly in an MR imaging or spectroscopy procedure, optionally after dilution or addition of further solution components, e.g. pH modifiers, complexing agents, etc. Such direct use may for example involve continuous infusion or alternatively injection or infusion of one or more dose units. Bolus injection is particularly interesting.

The whole process from beginning of hydrogenation to the delivery of the finished contrast agent in for example a syringe may conveniently be effected in less than 100 seconds, and more preferably in less than 10 seconds, which is substantially less than $T_1$ for the potentially interesting imaging nuclei.

Preferably, the surfaces contacted by the contrast agent during the process of the invention are substantially free of paramagnetic materials, e.g. made of glasses as used for hyperpolarized $^3He$ containment as discussed in WO99/17304 or gold or polymer, optionally deuterated. Surfaces contacting a non-aqueous solvent (e.g. acetone) should be acetone-resistant and valves may be magnetically controlled and provided with solvent resistant Teflon or silicone parts.

An apparatus suitable for producing contrast agent using the method according to the present invention will be described with reference to figure 1. Hydrogen ($^1H_2$) enriched in para-hydrogen is fed from the para-hydrogen source 200 into a reactor 210. A hydrogenation catalyst solution from a catalyst reservoir 230 and a hydrogenatable substrate solution from a substrate reservoir 220 are fed into the reactor 210. The liquid (contrast agent) settling in reactor 210 is transferred to a magnetic treatment unit 240, which essentially comprises a magnetic treatment chamber 245 for receiving a dosage of the contrast agent surrounded by means for producing the magnetic field 246 used in the field profile and a magnetic field screen 247 for shielding off external magnetic fields. The liquid contrast agent is thence transferred to finishing unit 250 for cleanup, quality control and possible addition of additives and solvent removal. The finishing unit may comprise an ion exchange column and a solvent removal chamber equipped with a spray nozzle. After the passage through the finishing units the contrast agent is delivered to, for example, a syringe for injection in a patient. Alternatively the contrast agent is stored for later usage. Alternatively, the magnetic treatment may be performed directly in the reactor 210, which then would be equipped with means for producing the magnetic field profile as well as magnetic shielding. Thus the need for a separate magnetic treatment chamber 245 is eliminated.

The low-field treatment (e.g. at fields below 50 $\mu$T) of the optimized field cycling profile typically needs to be performed in a magnetically shielded chamber. An effective magnetic shielding may be accomplished by using commercially available materials, e.g. $\mu$-metal in one or more layers and/or with by using compensating magnetic coils. In one embodiment of a low field treatment chamber, the magnetic field screen 247 is made from $\mu$-metal and consist of three concentric tubes, for example with diameters of 80, 25 and 12 mm, respectively.

$\mu$-Metal is slowly magnetized by external fields, especially in the vicinity of the large magnetic fields from the imaging magnets of the NMR-unit, and this has a degrading effect on the $\mu$-metal shielding. The shielding properties may be restored by a demagnetization process. For that purpose the low field treatment chamber is preferably equipped with demagnetization coils and circuitry to control the demagnetization process. As appreciated by those skilled in the art demagnetization may be performed in a number of ways. However, the present invention comprises a simple, yet effective, method and apparatus for demagnetization as follows:

The magnetic field screen 247 is provided with a demagnetization coil of about 30 turns, which together with a second coil of about 1000 turns and a dipolar capacitor of 250 $\mu$F forms a parallel resonance circuit with a resonance

frequency of approximately 50 Hz. The demagnetization process involves applying AC current of approximately 220V and 50 Hz to the circuit for approximately 1s and then letting the circuit decay for about 2s. The apparatus and method provides fast, effective and reproducible demagnetization and preferably should be performed on a daily basis.

The magnetic field means 246 necessary to produce the low and well-controlled magnetic field for the low field treatment is preferably realized by providing the interior of the magnetic treatment chamber 245 with an elongated resistive magnetic coil. The current through the coil, and hence the resulting magnetic field is precisely controllable, for example, by a computer controlled precision current supply. The coil is advantageously actively shielded for quicker response. In addition the treatment chamber 245 may be equipped with means for measuring the magnetic field, for example flux gates or Hall-probes in order to check the magnetic field strength. Other types of magnetic devices such as Helmoltz coils or superconducting magnets may also be used to produce the magnetic fields. The choice of magnetic set-up could depend on the desired field strength, the rate of change of the field or if it is required to be able to change the direction of the field. As an alternative the low field treatment may be achieved by transferring the contrast agent out of the earth magnetic field, into the μ-metal chamber, and back again in a controlled fashion. However, for more complex field cycling schemes the latter approach will be cumbersome.

Preferably, most of the functions are integrated in a PC environment, in particular the generation and control of the magnetic field pulse sequence.

To further increase the signal from the contrast agent, i.e. the polarisation of imaging nuclei, a careful analysis and optimization routine considering the effects of the field cycling on the nuclear spin states may advantageously be performed. The procedure will be exemplified with a molecule containing only one none-zero spin nucleus, $^{13}C$ with S=1/2, which is hydrogenated with para-hydrogen. It should be noted that the method according to the invention is not limited to this example.

Assume at first that $^{13}C$ is in its $\alpha$ state, corresponding to spin up. After being hydrogenated with para-hydrogen, which is described by the nuclear spin state $|\psi\rangle = \frac{1}{\sqrt{2}}\left(|\alpha\beta\rangle - |\beta\alpha\rangle\right)$, the combined spin state of the $^{13}C$ and the para-hydrogen nuclei is given by $|\psi_\alpha\rangle = |\psi\rangle \otimes |\alpha\rangle$.

This state can be written as a linear combination of the simple product states $|\alpha\beta\alpha\rangle$ and $|\beta\alpha\alpha\rangle$, where the last letter refers to the carbon spin state: $|\psi_\alpha\rangle = \frac{1}{\sqrt{2}}|\alpha\beta\alpha\rangle - \frac{1}{\sqrt{2}}|\beta\alpha\alpha\rangle$. The evolution of this state is given by:

$$|\psi_\alpha(t)\rangle = \exp(-itH)|\psi_\alpha(0)\rangle$$

where $H$ is the Hamiltonian. If the external magnetic field strength ($B_0$) that the system experiences is in the regime where the protons and carbon are weakly coupled, the state will after a time $t$ evolve to:

$$|\psi_\alpha(t)\rangle = C_1^\alpha(t)\cdot|\alpha\beta\alpha\rangle + C_2^\alpha(t)\cdot|\beta\alpha\alpha\rangle.$$

The probability of finding the system in state $|\alpha\beta\alpha\rangle$ or $|\beta\alpha\alpha\rangle$ is given by $\left|C_1^\alpha(t)\right|^2$ or $\left|C_2^\alpha(t)\right|^2$ respectively. If, on the other hand, the external field is so low that the protons and carbon are strongly coupled, the state is given by:

$$|\psi_\alpha(t)\rangle = C_1^\alpha(t)\cdot|\alpha\beta\alpha\rangle + C_2^\alpha(t)\cdot|\beta\alpha\alpha\rangle + C_3^\alpha(t)\cdot|\alpha\alpha\beta\rangle$$

and there is thus a finite probability of finding the system in state $|\alpha\alpha\beta\rangle$. This allows the carbon nucleus to change to state $|\beta\rangle$, by a mechanism that is not due to any relaxation process. This is one of the essential mechanisms by which the field cycling operates. In order to visualize the results graphically it is demonstrate what happens to a model system consisting of two protons (originating from para-hydrogen) and one carbon with the following scalar couplings: $J_{12}$ = 7.14 Hz, $J_{13}$ = 7.3 Hz and $J_{23}$ = 3.6 Hz. (The indices 1 and 2 refer to the protons and the index 3 to the carbon). The chemical shift difference between the protons is 1.64 ppm. This system resembles the spin system of compound (ignoring the deuterons)

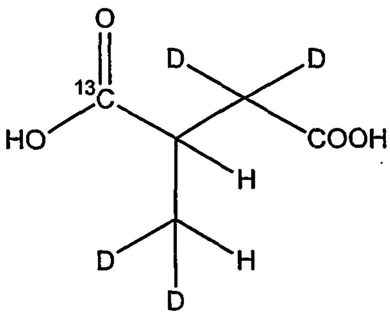

**1**

2-methylsuccinic acid (1-$^{13}$C, 1',1',2,2-$D_4$)

The time dependence of all three coefficients $\left|C_k^\alpha(t)\right|^2$ is depicted in figure 2. One can see that at 1 μT there is only a small contribution from the carbon beta state $|\alpha\alpha\beta\rangle$, but at 0.1 μT, when all three nuclei are strongly coupled, there is a large oscillating contribution from this state. Thus, after the sudden diabatic de-magnetization to low field, the system starts to evolve and obtains an increasing amount of $|\alpha\alpha\beta\rangle$.

Investigating a system where $^{13}C$ is in its β state, after hydrogenation the following state is obtained (after time *t*):

$$|\psi_\beta(t)\rangle = C_1^\beta(t)\cdot|\alpha\beta\beta\rangle + C_2^\beta(t)\cdot|\beta\alpha\beta\rangle + C_3^\beta(t)\cdot|\beta\beta\alpha\rangle$$

In analogy with the results above for $|\psi_\alpha(t)\rangle$, the contribution from the third state, $|\beta\beta\alpha\rangle$, is negligible if the carbon and the protons are weakly coupled. When the protons and the carbon are strongly coupled there will be a non-negligible contribution from the coefficient $C_3^\beta(t)$. The contribution from $|\alpha\alpha\beta\rangle$ is larger than the contribution from $|\beta\beta\alpha\rangle$. This lack of symmetry between the two cases is also an essential prerequisite for the field cycling to work.

The above descriptions of how the spin system evolves are for a constant field. The method according to the invention utilises a diabatic/adiabatic field cycling, which effects will now be demonstrated. To begin with the two cases where all carbons are either in their alpha or their beta state and all molecules are hydrogenated simultaneously will be treated separately. The evolution of the squared modulus of the coefficients $C_k^\alpha(t)$ can be used to calculate how the polarization changes during the field cycling. The carbon polarization in the first case is given by:

$$Pol_1 = P(\alpha\beta\alpha) + P(\beta\alpha\alpha) - P(\alpha\alpha\beta) = \left|C_1^\alpha(t)\right|^2 + \left|C_2^\alpha(t)\right|^2 - \left|C_3^\alpha(t)\right|^2$$

The polarization varies during the field cycling. Starting at 100 % (which was the initial assumption), the polarization oscillates and finally converges at approximately 20 %. For the second case the carbon polarization can be written as:

$$Pol_2 = P(\beta\beta\alpha) - P(\alpha\beta\beta) - P(\beta\alpha\beta) = \left|C_3^\beta(t)\right|^2 - \left|C_1^\beta(t)\right|^2 - \left|C_2^\beta(t)\right|^2$$

The behaviour of the polarization in this case is dramatically different from the previous one. Starting at -100 %, the polarization changes slightly but returns to a value close to -100 % after completing the field cycling. For a realistic situation (room temperature, etc.) the amounts of carbon in its alpha and beta states are approximately equal. Taking

the average of $Pol_1$ (20 %) and $Pol_2$ (-100 %), we arrive at a final polarization of approximately -40 %. In figure 3 a complete simulation including the effect of averaging due to non-simultaneous hydrogenation is shown. One interesting observation is that after 135 ms the polarization is below -60 %. If the field would be rapidly increased after 135 ms the polarization would be increased by a factor 1.5 compared to the adiabatic re-magnetization, and the field cycling procedure would be faster by a factor 7.4. Observations like this indicate that slight modifications in the field cycling profile yield dramatically increased degrees of polarisation in the so produced contrast agents.

Having thus described the contributions to the net polarisation from quantum mechanical principles it is possible to optimize the field cycling profile. One method is to represent the field profile by some adjustable parameters such as the coefficients of a polynomial, cubic spline or some other appropriate function. By varying the parameters in such representations almost any field cycling profile in question may be advantageously described. If a simulation program giving the net polarisation is run together with an optimization algorithm such as a simplex algorithm it is possible to optimize the parameters describing the field cycling profile.

The above discussion relates to a method of optimizing a field cycling profile according to the following algorithm, described with reference to figure 4:

400: Find the quantum mechanical wave functions describing the spin system of the combined para-hydrogen and imaging nuclei spin system. This gives the initial density matrix of the system. Use the field dependent Hamiltonian, most conveniently approximated as piecewise constant operators, to determine the evolution of the spin system. A suitable precision is obtained using 100-1000 operators, logarithmically spaced between 1 nT and 100 $\mu$T.

405: Calculate the net polarisation for the imaging nuclei given a field cycling profile.

410: Vary the parameters describing the field cycling profile according to an optimizing routine. Limitations in for example maximum/minimum magnetic field strength and maximum change rate, reflecting experimental constraints can be included.

415: Repeat steps 405-410 until the net polarisation reaches a maximum or a desired value.

420: Implement the field cycling profile in the above described apparatus for producing contrast agents.

425: Run the field cycling profile on each dose of contrast agent produced as described above.

The process of finding and implementing an optimized field cycling profile, steps 400-420, is typically performed if a new composition of the contrast agent is to be used, or if the magnetic conditions have been changed.

The simulation of the net polarization is typically performed by using a high-level programming language, such as MATLAB™, capable of handling the large matrices that are required. The matrix representations of the spin operators are generated in a suitable base, providing the spin density matrix and the matrix representation of the Hamiltonian. All subsequent evolutions of the spin system are then treated by multiplying matrices and functions of matrices. The polarization is finally obtained from the trace of the product of the final spin density matrix and the z-component of the relevant spin operator.

The optimization is typically performed by creating a function that uses the field cycling parameters as arguments and gives the polarization as the function value. Using standard optimization techniques, such as simplex algorithms, the optimal field cycling parameters can be determined. A suitable routine "fininsearch" is provided with MATLAB™.

The implementation and the realisation of the field cycling profile will depend on the design of the magnetic field treatment chamber of the apparatus, a few examples of which are given above. If a design utilizing different shielding of the earth magnetic field is used, the realisation of the desired field cycling profile may involve modifications to the design as well as moving the contrast agent in the shielded volume in a controlled manner. If the field treatment chamber is equipped with means for producing magnetic fields, for example, an elongated resistive magnetic coil, an elongated superconducting magnetic coil or Hehnholtz coils, the field cycling profile can be produced by varying the current in the coils. In all cases the control of the magnetic field is preferably computerised and the control system may further comprise means for measuring and calibrating the magnetic field such as Hall-elements. It may, for example, be necessary to utilize a feedback system with continuous measurement and adjustment of the magnetic field within the magnetic field treatment chamber in order to obtain the precision, both in field strength and field variation over time, necessary to achieve a field cycling profile with the required accuracy, typically $\pm$ 10 nT.

Figure 5 shows an example of an optimized field profile represented by a polynomial of degree 5 and in figure 6 the resulting polarization. The magnetic field within the magnetic field treatment chamber is initially at approximately the earth magnetic field ($1x10^{-4}$ T). In a first step the field is extremely rapidly reduced (i.e. in less than 1ms) to a field in the order of $1x10^{-7}$ T. During a next step the field is further lowered to approximately $4x10^{-8}$ T during a period of 10

ms. The field is then gradually increased up to approximately 1x10$^{-4}$ T over approximately 300 ms. In figure 6 the resulting net polarisation is shown. During the first 30 ms the polarization quickly drops to below -90 % and after an additional 50 ms the polarization has settled at its final value of- 92.4 %. The field cycling profile used gives a high polarization after an exceptionally short field cycling time. The following difference to the prior art methods, described in for example WO 00171166 should be noted: in the prior art methods the field is suddenly reduced and followed by a comparably slow adiabatic re-magnetisation prevailing for up to some tens of seconds, the field increase preferably follows an exponential curve. In the method according to the present invention a controlled reduction of the field is followed by a comparably rapid increase of the field, giving a non-adiabatic (diabatic) re-magnetisation. The complete field cycling process can be performed in typically less than 0.3 s and still give a polarisation at the same level or higher than the prior art methods.

For many of the molecules making up the functional part of the contrast agent a low magnetic field treatment according to the following steps below would be suitable. The determination of the parameters describing the precise field strength, duration of the steps etc, resulting in an (nearly) optimized field cycling profile, should preferably be done with the above described method of finding a field cycling profile. The low magnetic field treatment including the optimized field profile will be described with reference to FIG. 7 and comprises the steps of:

700: The dose or part of the dose of contrast agent is placed in the magnetic treatment chamber, which is well shielded from the earth magnetic field as well as other external magnetic fields. A magnetic field in the order of the earth magnetic field should be present in the chamber when the sample is placed therein.

705: The contrast agent is subjected to a precisely controlled field cycling profile (field vs. time profile). The field cycling profile comprises cycling of the field from a field in the order of the earth magnetic field to a low field, in the order of 1-100 nT, and back again according to an optimised profile preferably derived with the above described method. The profile may contain several maxima and minima. The complete profile should preferably be shorter than 2 seconds, more preferably shorter than 1 second, even more preferably shorter than 500 ms and most preferably as short as 100 ms or less. An optimize profile may typically comprise the steps of:

705.1: Rapidly reducing the field from approximately the earth magnetic field to a very low field (1-100 nT) in 1 ms (1x10$^{-3}$ seconds) or less. A longer reduction time will significantly impair the result.

705.2: Increasing the field from the very low field to approximately the earth magnetic field. The increase should be slow compared to the preceding decrease (orders of magnitude slower, typically more than 100 times slower) and arranged to give a give a non-adiabatic (diabatic) re-magnetisation.

710: The dose, or part of the dose of contrast agent is removed from the low magnetic field treatment chamber and the remaining (chemical) process steps are performed prior to injection.

The method is preferably implemented by means of a computer program product comprising the software code means for performing the steps of the method by controlling parts of the apparatus according to the invention. The computer program product is typically executed on the computer controlling the apparatus. The computer program is loaded directly or from a computer usable medium, such as a floppy disc, a CD, the Internet etc.

From the invention thus described, it will be obvious that the invention may be varied in many ways. Such variations are not to be regarded as a departure from the inventive concept, and all such modifications as would be obvious to one skilled in the art are intended for inclusion within the scope of the following claims.

## Claims

**1.** A method for producing of MR contrast agent, the method comprising the steps of

- *obtaining* (100) a solution in a solvent of a hydrogenatable, unsaturated substrate compound and a catalyst for the hydrogenation of a substrate compound, wherein the substrate compound comprises imaging nuclei;
- *hydrogenating* (105) the substrate with hydrogen gas ($H_2$) enriched in para-hydrogen (p-$^1H_2$) to form a hydrogenated contrast agent;
- *exposing* (110: 705) the contrast agent to a magnetic field cycling profile adapted for enhancing the contrasting effects of the contrast agent adapted for use in an MR application, the magnetic field cycling profile comprising an initial decrease of the magnetic field followed by at least one increase of the magnetic field, said at least one increase arranged to provide a non-adiabatic (diabatic) re-magnetisation of the contrast agent.

2. The method according to claim 1 **wherein** the method further comprises the steps of:

- *placing* (700) a dose or part of a dose of the contrast agent in a magnetically shielded magnetic treatment chamber, with a magnetic field in the order of the earth magnetic field present within the magnetic treatment chamber at the introduction of said dose of contrast agent into said magnetic treatment chamber;
- *removing* (710) the dose or part of the dose of the contrast agent from the magnetic treatment chamber.

3. The method according to claim 2 **wherein** said initial decrease of the magnetic field according to the field cycling profile is from a field in the order of the earth magnetic field to a low field in the order of 1-100 nT.

4. The method according to claim 2 **wherein** said initial decrease of the magnetic field is performed in less than 10 ms ($10x10^{-3}$ seconds) (705.1).

5. The method according to claim 2 **wherein** said initial decrease of the magnetic field is performed in less than 1 ms ($1x10^{-3}$ seconds) (705.1).

6. The method according to claims 3 or 1 **wherein** said at least one increase (705.2) of the magnetic field according to the field cycling profile is substantially slower than the initial decrease of the magnetic field.

7. The method according to claim 6 **wherein** said at least one increase (705.2) of the magnetic field according to the field cycling profile is at least ten times slower than the initial decrease of the magnetic field.

8. The method according to claim 7 **wherein** a complete field cycling profile is performed in less than 2 seconds.

9. The method according to claim 7 **wherein** a complete field cycling profile is performed in less than 100 ms ($1x10^{-3}$ seconds).

10. The method according to any of claims 1 to 9 **wherein** the field cycling profile is described with a set of field cycling parameters and said field cycling parameters are determined by a process comprising the steps of:

- *finding* (400) the quantum mechanical density operator describing the initial spin order of the combined para-hydrogen and imaging nuclei spin system;
- *simulating* the polarisation for the imaging nuclei given a field cycling profile;
- *varying* the field cycling parameters according to an optimizing routine;
- *repeating* the simulating step and varying the field cycling parameters until the net polarisation reaches a maximum value or a desired value.

11. The method according to claim 2 **wherein** the method further comprises an optional step of demagnetizing the magnetic field screen (247) of the magnetic treatment chamber (246), utilizing a demagnetization circuit comprising of a demagnetization coil arranged around the magnetic shield, which together with a second coil and a dipolar capacitor forms a parallel resonance circuit, the demagnetization step comprises:

- *applying* an AC current to a demagnetization circuit for approximately 1 s;
- *removing* the AC current and then letting the circuit decay for approximately 2s.

12. A computer program product directly loadable into the internal memory of a processing means within a processing unit for controlling the method and apparatus for producing MR contrast agent, comprising the software code means adapted for controlling the steps of any of the claims 1 to 11.

13. A computer program product stored on a computer usable medium, comprising a readable program adapted for causing a processing means, in a processing unit for controlling the method and apparatus for producing MR contrast agent, to control an execution of the steps of any of the claims 1 to 11

14. Apparatus for producing MR contrast agent, the apparatus comprising a magnetic field screen (247) arranged around a magnetic treatment chamber (246) adapted for magnetic treatment of the contrast agent, **characterized by** a demagnetization circuit adapted form demagnetization of the magnetic field screen (247), which demagnetization circuit comprises of:

a demagnetization coil of about 30 turns arranged on the magnetic shield;
a second coil of about 1000 turns;
a dipolar capacitor of approximately 250 μF, which together with the second coil forms a parallel resonance circuit connected to the demagnetization coil, which resonance circuit is arranged to have a resonance frequency of approximately 50 Hz.

**15.** Apparatus for producing MR contrast agent according to claim 14, the apparatus comprising storing means for storing enriched hydrogen, wherein the storing means are essentially free from para-hydrogen relaxing material.

**16.** Apparatus for producing MR contrast agent according to claim 15, wherein the storing means are made of aluminum or carbon-fiber reinforced epoxy.

**Patentansprüche**

**1.** Verfahren zum Herstellen eines MR-Kontrastmittels, wobei das Verfahren die Schritte umfasst:

- Erhalten (100) einer Lösung einer hydrierbaren ungesättigten Substratverbindung und eines Katalysators für die Hydrierung einer Substratverbindung in einem Lösungsmittel, wobei die Substratverbindung Bildgebungskerne umfasst;
- Hydrieren (105) des Substrats mit Wasserstoffgas ($H_2$), das in para-Wasserstoff (p-$^1H_2$) angereichert ist, um ein hydriertes Kontrastmittel zu bilden;
- Aussetzen (110;705) des Kontrastmittels einem Magnetfeldkreislaufprofil, das zum Verstärken der Kontrastierungseffekte des Kontrastmittels angepasst ist, das zur Verwendung in einer MR-Anwendung angepasst ist, wobei das Magnetfeldkreislaufprofil eine anfängliche Abnahme des Magnetfelds gefolgt von mindestens einem Anstieg des Magnetfelds umfasst, wobei der mindestens eine Anstieg gestaltet ist, um eine nicht-adiabatische (diabatische) Remagnetisierung des Kontrastmittels zu liefern.

**2.** Verfahren nach Anspruch 1, wobei das Verfahren weiter umfasst die Schritte:

- Anordnen (700) einer Dosis oder eines Teils einer Dosis des Kontrastmittels in einer magnetisch abgeschirmten Kammer zur magnetischen Behandlung, wobei ein Magnetfeld in der Größenordnung des Erdmagnetfeldes innerhalb der Kammer zur magnetischen Behandlung beim Einführen der Dosis des Kontrastmittels in die Kammer zur magnetischen Behandlung vorhanden ist;
- Entfernen (710) der Dosis oder eines Teils der Dosis des Kontrastmittels aus der Kammer zur magnetischen Behandlung.

**3.** Verfahren nach Anspruch 2, wobei die anfängliche Abnahme des Magnetfelds gemäß dem Feldkreislaufprofil stattfindet von einem Feld in der Größenordnung des Erdmagnetfelds zu einem niedrigen Feld in der Größenordnung von 1-100 nT.

**4.** Verfahren nach Anspruch 2, wobei die anfängliche Abnahme des Magnetfelds in weniger als 10 ms ($10 \times 10^{-3}$ Sekunden) (705.1) ausgeführt wird.

**5.** Verfahren nach Anspruch 2, wobei die anfängliche Abnahme des Magnetfelds ausgeführt wird in weniger als 1 ms ($1 \times 10^{-3}$ Sekunden) (705.1).

**6.** Verfahren nach den Ansprüchen 3 oder 1, wobei mindestens ein Anstieg (705.2) des Magnetfelds gemäß dem Feldkreislaufprofil im wesentlichen langsamer ist als die anfängliche Abnahme des Magnetfelds.

**7.** Verfahren nach Anspruch 6, wobei mindestens ein Anstleg (705.2) des Magnetfelds gemäß dem Feldkreislaufprofil mindestens zehn Mal langsamer ist als die anfängliche Abnahme des Magnetfelds.

**8.** Verfahren nach Anspruch 7, wobei ein vollständiges Feldkreislaufprofil in weniger als zwei Sekunden ausgeführt wird.

**9.** Verfahren nach Anspruch 7, wobei ein vollständiges Feldkreislaufprofil ausgeführt wird in weniger als 100 ms ($1 \times 10^{-3}$ Sekunden).

**10.** Verfahren nach einem der Ansprüche 1 bis 9, wobei das Feldkreislaufprofil beschrieben wird mit einem Satz von Feldkreislaufparametern und die Feldkreislaufparameter bestimmt werden durch einen Prozess mit den Schritten:

- Auffinden (400) des quantenmechanlschen Dichteoperators, der die anfängliche Spinordnung des kombinierten para-Wasserstoff- und Bildgebungskerne-Spinsystems beschreibt;
- Simulieren der Polarisation für die Bildgebungskerne bei einem gegebenen Feldkreislaufprofil;
- Variieren der Feldkreislaufparameter gemäß einer Optimierungsroutine;
- Wiederholen des Simulierschrittes und Variieren der Feldkreislaufparameter, bis die Nettopolarisation einen Maximalwert oder einen erwünschten Wert erreicht.

**11.** Verfahren nach Anspruch 2, wobei das Verfahren weiter umfasst einen optionalen Schritt des Entmagnetisierens des Magnetfeldschirms (247) der Kammer zur magnetischen Behandlung (246), unter Nutzen einer Entmagnetisierungsschaltung, umfassend eine Entmagnetisierungsspule, die um den Magnetschirm angeordnet ist, welche zusammen mit einer zweiten Spule und einem dipolaren Kondensator eine Parallelresonanzschaltung bildet, wobei der Entmagnetisierungsschritt umfasst:

- Anwenden eines AC-Stromes auf eine Entmagnetisierungsschaltung für annähernd 1 s;
- Entfernen des AC-Stromes und dann Abklingenlassen der Schaltung für annähernd 2 s.

**12.** Computerprogrammprodukt, das direkt in den internen Speicher eines Verarbeitungsmittels innerhalb einer Verarbeitungseinheit ladbar ist zum Steuern des Verfahrens und der Vorrichtung zum Herstellen eines MR-Kontrastmlttels, umfassend das Softwarecodemittel, das zum Steuern der Schritte nach einem der Ansprüche 1 bis 11 angepasst ist.

**13.** Computerprogrammprodukt, das auf einem Computer-nutzbaren Medium gespeichert ist, umfassend ein lesbares Programm, das zum Veranlassen eines Verarbeitungsmittels angepasst ist, in einer Verarbeitungseinheit zum Steuern des Verfahrens und der Vorrichtung zum Herstellen eines MR-Kontrastmittels, eine Ausführung der Schritte nach einem der Ansprüche 1 bis 11 zu steuern.

**14.** Vorrichtung zum Herstellen eines MR-Kontrastmittels, wobei die Vorrichtung einen Magnetfeldschirm (247) umfasst, der um eine Kammer (246) zur magnetischen Behandlung angeordnet ist, die für eine magnetische Behandlung des Kontrastmittels angepasst ist, **gekennzeichnet durch** eine Entmagnetisierungsschaltung, die zum Entmagnetisieren des Magnetfeldschirms (247) angepasst ist, wobei die Entmagnetisierungsschaltung umfasst:

eine Entmagnetisierungsspule von ungefähr 30 Windungen, die um die magnetische Abschirmung angeordnet ist;
eine zweite Spule von ungefähr 1000 Windungen;
einen dipolaren Kondensator von ungefähr 250 μF, der zusammen mit der zweiten Spule eine parallele Resonanzschaltung bildet, die mit der Entmagnetisierungsspule verbunden ist, wobei die Resonanzschaltung angeordnet ist, um eine Resonanzfrequenz von annähernd 50 Hz zu besitzen.

**15.** Vorrichtung zum Herstellen eines MR-Kontrastmittels nach Anspruch 14, wobei die Vorrichtung umfasst Speichermittel zum Speichern von angereichertem Wasserstoff, wobei die Speichermittel im wesentlichen frei von para-Wasserstoff-relaxierendem Material sind,

**16.** Vorrichtung zum Herstellen eines MR-Kontrastmittels nach Anspruch 15, wobei die Speichermittel aus Aluminium oder Kohlenstofffaser-verstärktem Epoxy erzeugt sind.

**Revendications**

**1.** Procédé de production d'un agent de contraste à résonance magnétique (RM), le procédé comprenant les étapes de :

obtention (100) d'une solution dans un solvant d'un composé de substrat insaturé pouvant être hydrogéné et d'un catalyseur afin d'assurer l'hydrogénation d'un composé de substrat, dans lequel le composé de substrat comprend des noyaux d'imagerie ;
hydrogénation (105) du substrat avec du gaz hydrogène ($H_2$) enrichi en parahydrogène (p-$^1H_2$) afin de former un agent de contraste hydrogéné ;
exposition (110: 705) de l'agent de contraste à un profil de variation cyclique de champ magnétique adapté afin

d'améliorer les effets de contraste de l'agent de contraste adapté à une utilisation dans une application de RM, le profil de variation cyclique de champ magnétique comprenant une diminution initiale du champ magnétique, suivie par au moins une augmentation du champ magnétique, ladite au moins une augmentation étant mise en oeuvre afin de produire une remagnétisation non adiabatique (avec échange de chaleur) de l'agent de contraste.

**2.** Procédé selon la revendication 1, dans lequel le procédé comprend, en outre, les étapes de :

mise en place (700) d'une dose ou partie d'une dose de l'agent de contraste dans une chambre de traitement magnétique blindée magnétiquement, un champ magnétique de l'ordre du champ magnétique terrestre étant présent à l'intérieur de la chambre de traitement magnétique à l'introduction de ladite dose d'agent de contraste dans ladite chambre de traitement magnétique ;
retrait (710) de la dose ou partie de la dose de l'agent de contraste de la chambre de traitement magnétique.

**3.** Procédé selon la revendication 2, dans lequel ladite diminution initiale du champ magnétique selon le profil de variation cyclique de champ commence à partir d'un champ de l'ordre de grandeur du champ magnétique terrestre jusqu'à un champ faible de l'ordre de 1 à 100 nT.

**4.** Procédé selon la revendication 2, dans lequel ladite diminution initiale du champ magnétique est réalisée en moins de 10 ms ($10x10^{-3}$ seconde) (7D5.1).

**5.** Procédé selon la revendication 2, dans lequel ladite diminution initiale du champ magnétique est réalisée en moins de 1 ms ($1x10^{-3}$ seconde) (705.1).

**6.** Procédé selon les revendications 3 ou 1, dans lequel ladite au moins une augmentation (705.2) du champ magnétique selon le profil de variation cyclique de champ est sensiblement plus lente que la diminution initiale du champ magnétique.

**7.** Procédé selon la revendication 6, dans lequel ladite au moins une augmentation (705.2) du champ magnétique selon le profil de variation cyclique de champ est au moins dix fois plus lente que la diminution initiale du champ magnétique.

**8.** Procédé selon la revendication 7, dans lequel le profil de variation cyclique de champ complet est réalisé en moins de 2 secondes.

**9.** Procédé selon la revendication 7, dans lequel le profil de variation cyclique de champ complet est réalisé en moins de 100 ms ($1x10^{-3}$ seconde).

**10.** Procédé selon l'une quelconque des revendications 1 à 9, dans lequel le profil de variation cyclique de champ est défini avec un jeu de paramètres de variation cyclique de champ et lesdits paramètres de variation cyclique de champ sont déterminés par un procédé comprenant les étapes de :

recherche (400) de l'opérateur de densité de mécanique quantique décrivant l'ordre de spin initial du système de spin du parahydrogène et des noyaux d'imagerie associés ;
simulation de la polarisation des noyaux d'imagerie donnés dans un profil de variation cyclique de champ ;
modification des paramètres de variation cyclique de champ en fonction d'un programme d'optimisation ;
répétition de l'étape de simulation et modification des paramètres de variation cyclique de champ jusqu'à ce que la polarisation nette atteigne une valeur maximum ou une valeur désirée.

**11.** Procédé selon la revendication 2, dans lequel le procédé comprend, en outre, une étape optionnelle de démagnétisation de l'écran de blindage de champ magnétique (247) de la chambre de traitement magnétique (246), en utilisant un circuit de démagnétisation comprenant une bobine de démagnétisation agencée autour du champ magnétique qui, ensemble avec une seconde bobine et un condensateur dipolaire, forme un circuit résonant parallèle, l'étape de démagnétisation comprend :

l'application d'un courant alternatif sur un circuit de démagnétisation pendant approximativement 1 s ;
la suppression du courant alternatif et ensuite, la relaxation du circuit pendant approximativement 2 s.

**12.** Produit formant programme informatique pouvant être chargé directement dans la mémoire Interne d'un moyen de

traitement à l'intérieur d'une unité de traitement afin d'assurer la commande du procédé et du dispositif de production d'agent de contraste RM, comprenant les moyens de code logiciel adaptés afin de commander les étapes de l'une quelconque des revendications 1 à 11.

**13.** Produit formant programme informatique mémorisé sur un support pouvant être utilisé sur ordinateur, comprenant un programme pouvant être lu, adapté afin d'amener un moyen de traitement, dans une unité de traitement, à commander le procédé et le dispositif de production d'agent de contraste RM, de manière à commander une exécution des étapes selon l'une quelconque des revendications 1 à 11.

**14.** Dispositif de production d'agent de contraste à résonance magnétique (RM), le dispositif comprenant un écran de blindage de champ magnétique (247) agencé autour d'une chambre de traitement magnétique (246) adaptée afin d'assurer un traitement magnétique de l'agent de contraste, **caractérisé par** un circuit de démagnétisation adapté de manière à assurer la démagnétisation de l'écran de blindage de champ magnétique (247), lequel circuit de démagnétisation comprend :

une bobine de démagnétisation de 30 spires environ, agencée dans le champ magnétique ;
une seconde bobine de 1000 spires environ ;
un condensateur bipolaire de 250 μF approximativement, qui, ensemble avec la seconde bobine, forme un circuit résonant parallèle raccordé à la bobine de démagnétisation, lequel circuit résonant est agencé de manière à présenter une fréquence de résonance approximativement égale à 50 Hz.

**15.** Dispositif de production d'agent de contraste RM selon la revendication 14, le dispositif comprenant des moyens de stockage afin de stocker de l'hydrogène enrichi, dans lequel les moyens de stockage sont sensiblement exempts de matériaux relaxant pour le parahydrogène.

**16.** Dispositif de production d'agent de contraste selon la revendication 15, dans lequel les moyens de stockage sont réalisés en aluminium ou en époxy renforcé par des fibres de carbone.

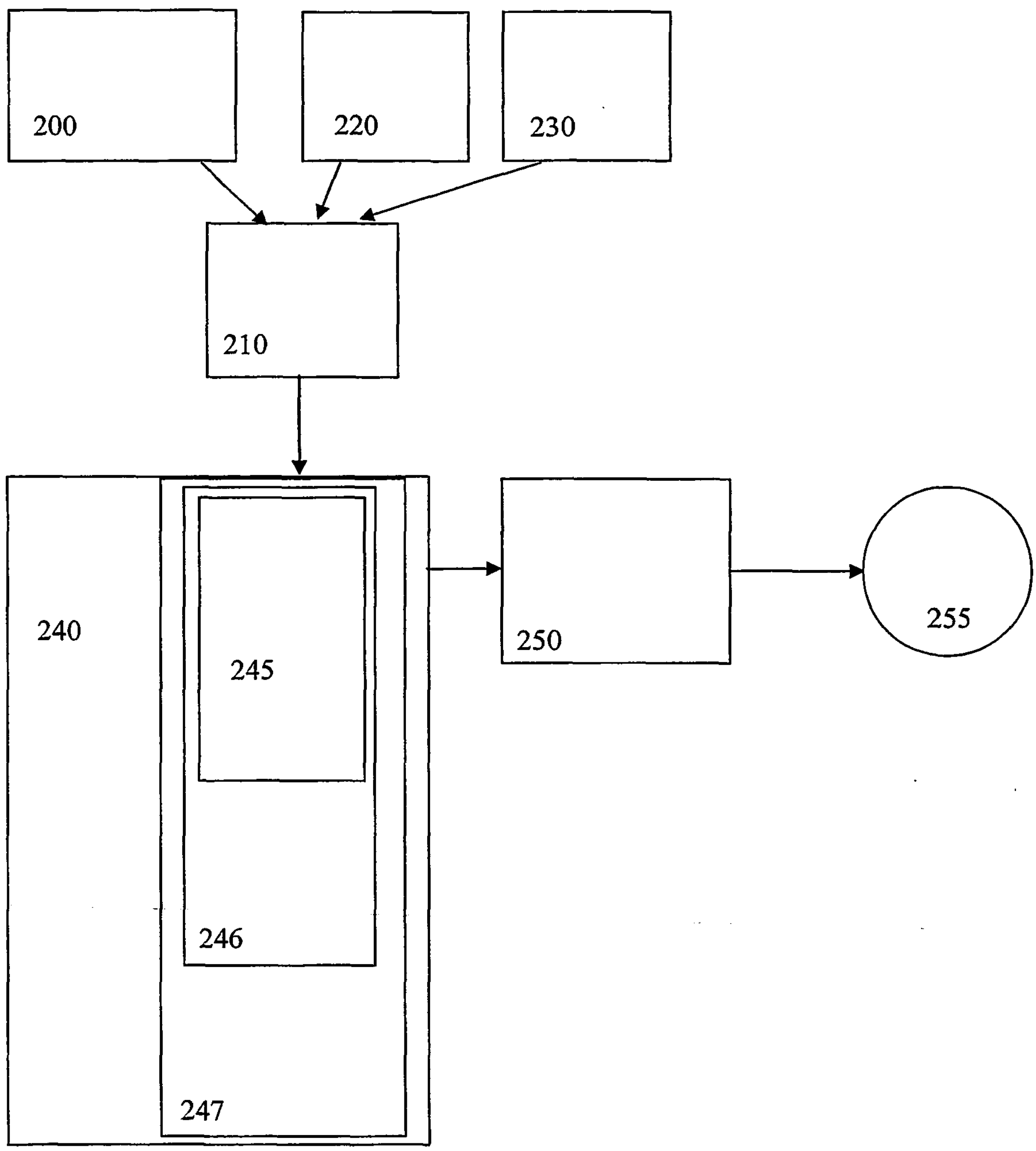


*FIG. 1*

FIG. 2a)

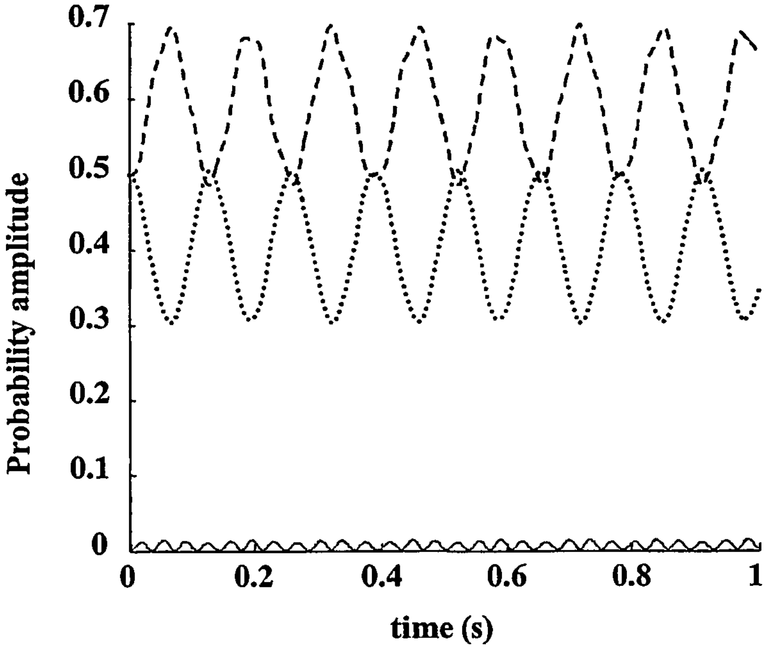
0.7
0.6
0.5
0.4
0.3
0.2
0.1
0
Probability amplitude
0
0.2
0.4
0.6
0.8
1
time (s)

FIG. 2b)

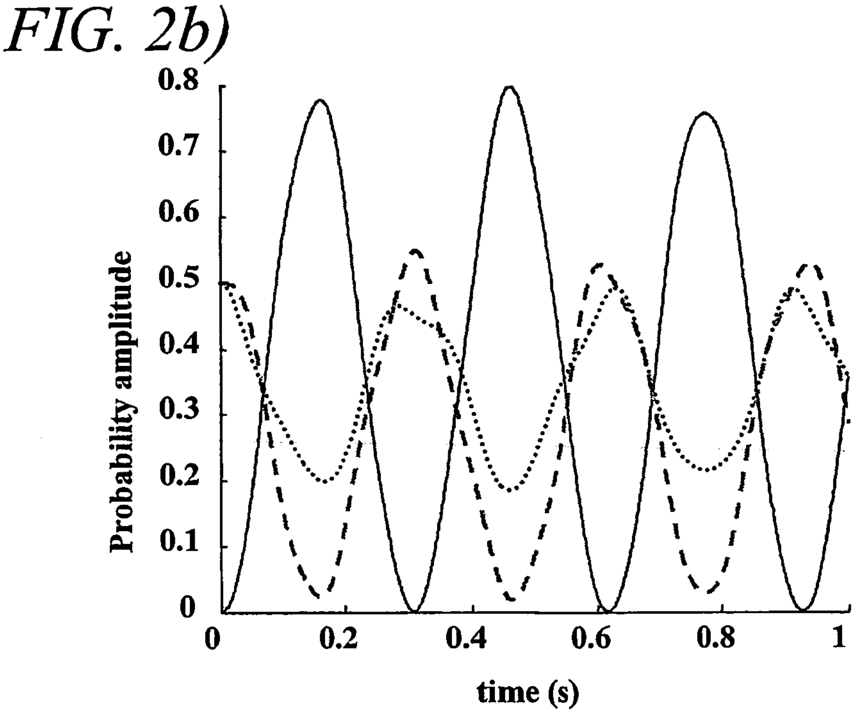
0.8
0.7
0.6
0.5
0.4
0.3
0.2
0.1
0
Probability amplitude
0
0.2
0.4
0.6
0.8
1
time (s)

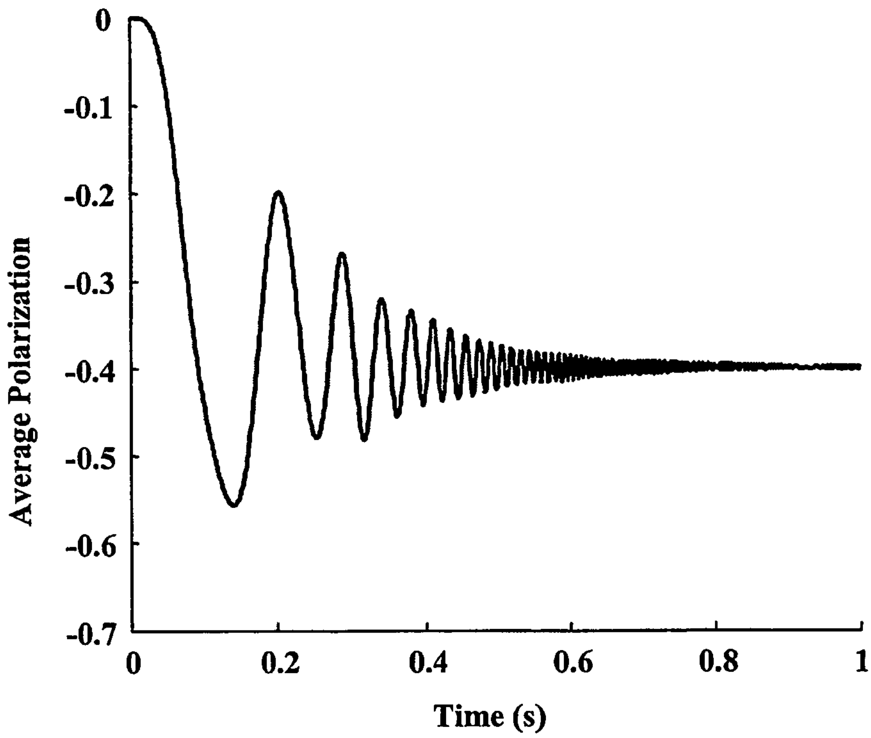
0
-0.1
-0.2
-0.3
-0.4
-0.5
-0.6
-0.7
Average Polarization
0
0.2
0.4
0.6
0.8
1
Time (s)

*FIG. 3*

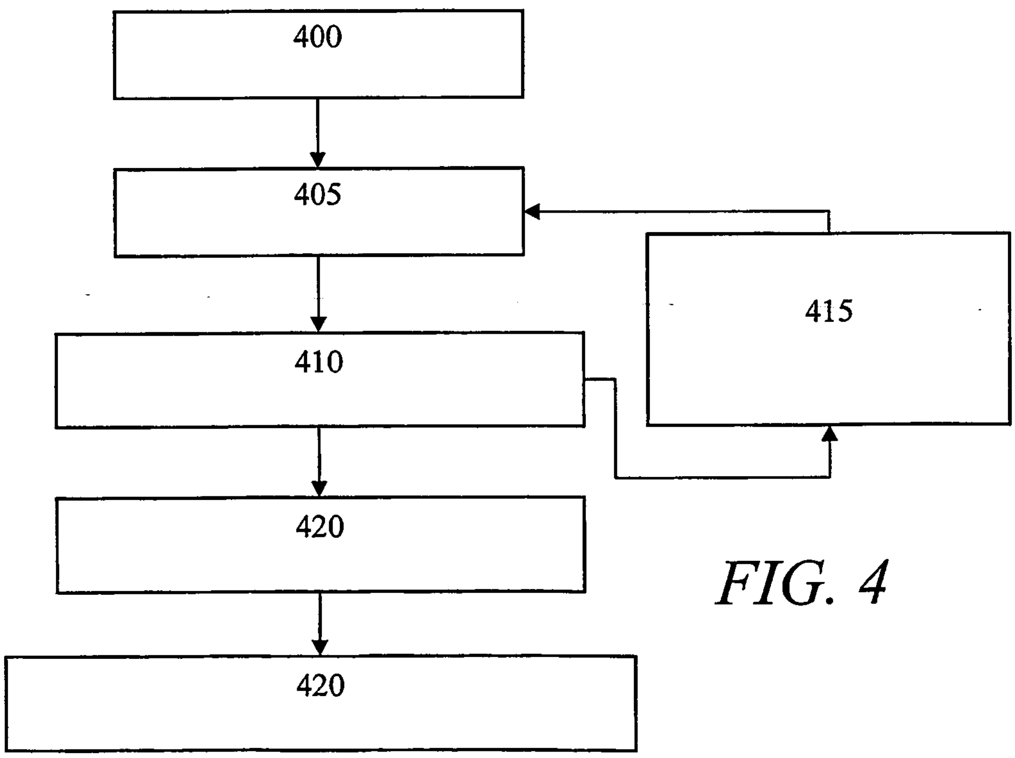
400
405
415
410
420
420

*FIG. 4*

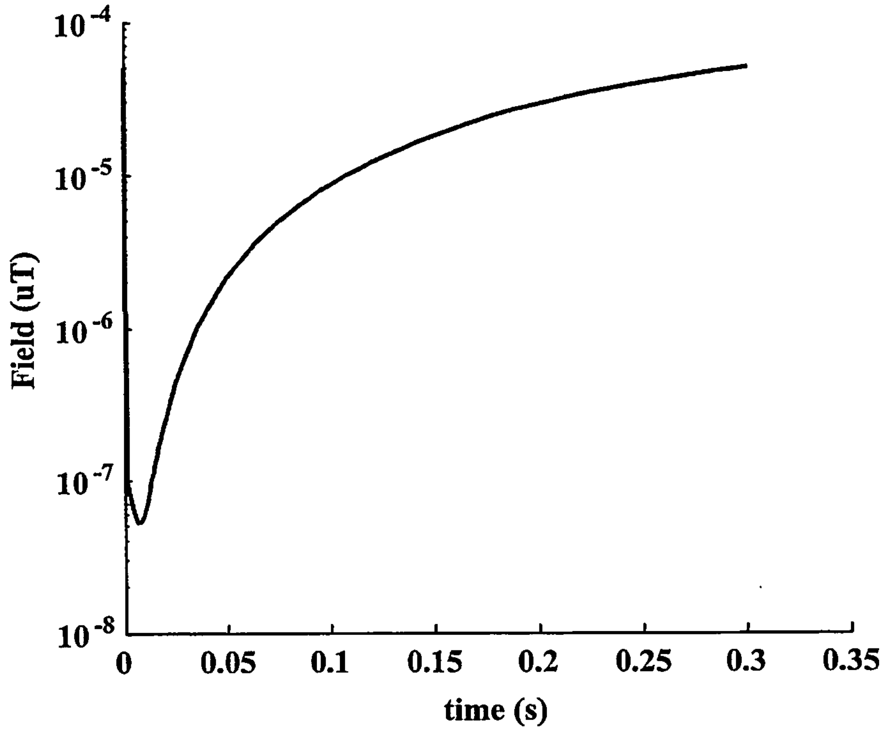
10^-4
10^-5
10^-6
10^-7
10^-8
Field (uT)
0
0.05
0.1
0.15
0.2
0.25
0.3
0.35
time (s)

*FIG. 5*

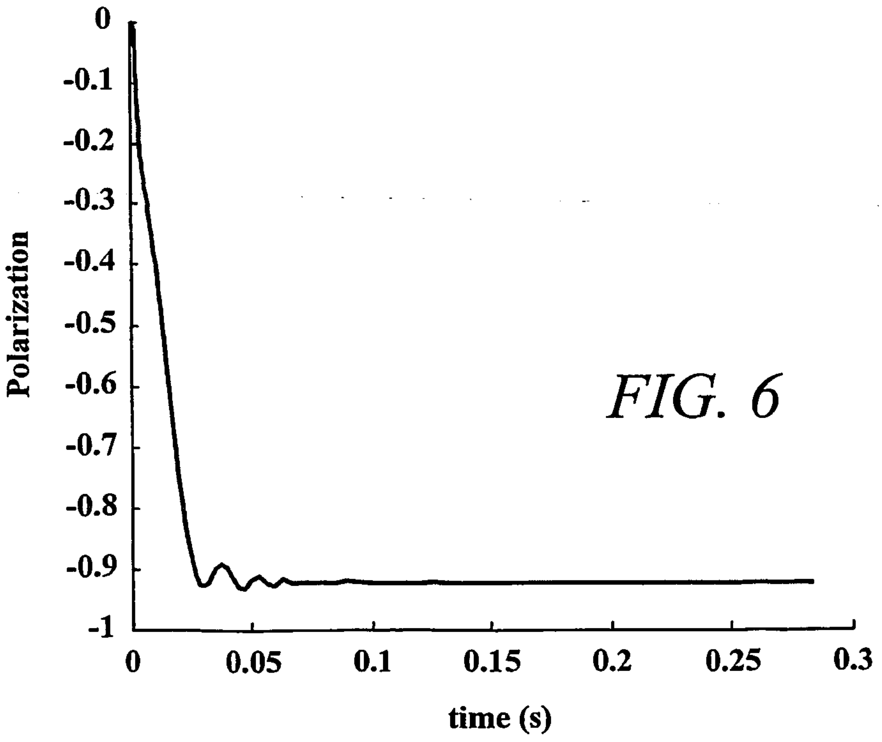
0
-0.1
-0.2
-0.3
-0.4
-0.5
-0.6
-0.7
-0.8
-0.9
-1
Polarization
0
0.05
0.1
0.15
0.2
0.25
0.3
time (s)

*FIG. 6*

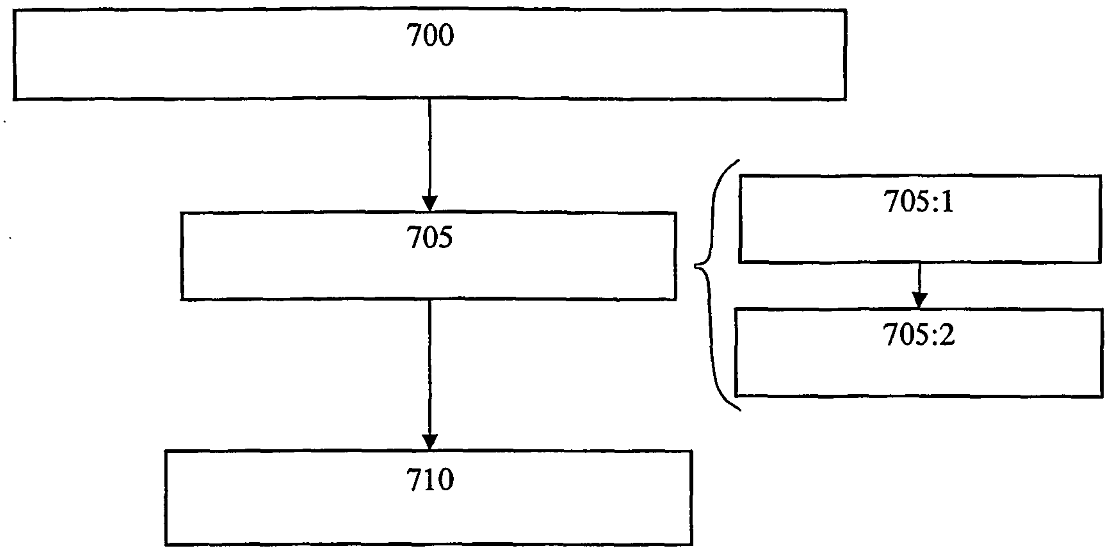


*FIG. 7*